# EUROPEAN PATENT APPLICATION

(11) **EP 3 861 931 A1**
(43) Date of publication of application: **11.08.2021**
(21) Application number: 20155844.2
(22) Date of filing: 06.02.2020
(51) Int. Cl.: A61B 5/055, A61B 5/08, G01R 33/385

(54) **A BREATHING GUIDANCE SYSTEM AND A MAGNETIC RESONANCE IMAGING SYSTEM USING THE BREATHING GUIDANCE SYSTEM**

(71) Applicant: Koninklijke Philips N.V., 5656 AG Eindhoven (NL)
(72) Inventor: VAN EE, Raymond, 5656 AE Eindhoven (NL); LEUFKENS, Timmy Robertus Maria, 5656 AE Eindhoven (NL); WESTERINK, Joanne Henriëtte Desirée Monique, 5656 AE Eindhoven (NL); MATTERS, Marco, 5656 AE Eindhoven (NL); DENISSEN, Adrianus Johannes Maria, 5656 AE Eindhoven (NL); HUIJBERS, Willem, 5656 AE Eindhoven (NL)
(74) Representative: Philips Intellectual Property & Standards

(57) **Abstract**

A breathing guidance system is provided for guiding the breathing of a user during a magnetic resonance imaging procedure. A target breathing rate is determined for the user which is in synchronism with the sounds, such as clicking sounds, generated by the MRI scanner. In this way, an improved signal to noise ratio is obtained for the scanned image by controlling the breathing of the user to be regular.

## Description

### FIELD OF THE INVENTION

This invention relates to magnetic resonance imaging systems.

### BACKGROUND OF THE INVENTION

Magnetic resonance imaging is widely used for the diagnosis and monitoring of medical conditions. MRIs employ powerful magnets which produce a strong magnetic field that forces protons in the body to align with that field. When a radiofrequency current is then pulsed through the patient, the protons are stimulated, and spin out of equilibrium, straining against the pull of the magnetic field.

When the radiofrequency field is turned off, the MRI sensor is able to detect the energy released as the protons realign with the magnetic field. The time it takes for the protons to realign with the magnetic field, as well as the amount of energy released, changes depending on the environment and the chemical nature of the molecules. Physicians are able to tell the difference between various types of tissues based on these magnetic properties.

MRI scans are used to image anatomical structure. Functional magnetic resonance imaging (fMRI) of a body part is used to image metabolic function, and is based upon the magnitude of the variation of oxygen in the blood that the body part consumes. The consumed oxygen provides information about tissue-type and functionality.

The variation of oxygen in the blood that a body part consumes is for example measured during a functional MRI scan by first causing alternations of a very strong magnetic field. A disadvantage of making such magnetic field alternations is that they are accompanied by loud auditory clicks. The clicks are caused by the vibration of the metal coils in the machine due to rapidly alternating pulses of electricity.

The clicking sound of a magnetic resonance imaging bore during scanning is usually experienced as annoying, and indeed measures have been proposed to suppress or mask these sounds.

A particular issue with functional MRI is that the oxygen level is not only influenced by the functionality and tissue type of the body part, but the level can also be influenced by breathing.

When performing an MRI scan, it is preferable that the (variation in) oxygen level of the blood is known, as much as possible, at all times during the scanning period in order to improve the information content and interpretation of the image.

Variation in breathing rate of a patient during the scanning period will contribute to a decreased signal to noise ratio. A constant breathing rate of a patient during the scanning period will conversely contribute to an increased signal to noise ratio. Although it is impossible to keep the blood-oxygen level entirely constant during the scanning period, the best measure that can be taken to to prevent a decrease in signal to noise ratio is to ensure that the patient's breathing rate is as constant, and predictable, as possible.

There is a need for an improved way to improve a scan quality by synchronizing the scan procedure with the respiration cycle.

### SUMMARY OF THE INVENTION

The invention is defined by the claims.

According to examples in accordance with an aspect of the invention, there is provided a breathing guidance system for guiding the breathing of a user during a magnetic resonance imaging procedure, comprising:
an input for receiving rhythm information relating to sounds generated by a magnetic resonance imaging system during a particular scanning procedure;
a breathing sensor for sensing breathing of the user and for determining a reference breathing rate of the user;
a processor for determining a target breathing rate for the user based on the rhythm information and the reference breathing rate, the target rate being in synchronism with the generated sounds; and
an output device for providing a stimulus for guiding the user to breathe at the target breathing rate.

The invention is based on the realization that the rhythmic clicking sounds of an MRI scanner can be used as part of a breathing guidance system, to control the breathing rate of a patient, and thereby enable an improved scan. The clicking sound of an MRI scanner bore during scanning has a very regular predictable rhythm over time. By linking the clicking sound to the breathing rate, positive use is made of the clicking sound in generating the desired uniform breathing pattern.

The target breathing rate may be first breathing rate to which the user is guided, but there may be multiple targets in sequence, for example if it is desired to guide the user gradually to slow their breathing. Thus, the target breathing rate at any particular time may or may not be a final target breathing rate. The breathing guidance could also present a gradual change from the current breathing to the target breathing rate so that it is easier for the patient to adopt the new target breathing rate.

The guidance aims to provide a predictable and regular breathing of the user.

The output device functions as a sensory modality with a rhythm that matches the rhythm of the clicking sound (typically synchronized with a sequence of multiple clicks) of the MI scanner bore. This provides guidance to assist the patient in breathing with a constant rhythm that matches the clicking sounds of the MRI scanner bore.

The combination of the clicking sounds and the output device stimulus provides rhythmically synchronous multimodal stimulus signals. It has been found that such multimodal stimulation provides improved perception of information and thus makes following the guidance more natural and effortless.

In this way, the MRI-scan procedure does not have to be adjusted, as it is the patient who adapts to an imposed breathing rate.

The output device for example comprises a display device such as a visual bar, or disk, that changes in size to impose a breathing rate (for example a large visual image corresponds to a full lung at the end of inhalation and a small visual image correspond to an empty lung at the end of exhalation). The patient is instructed to breathe with the same rhythm as the variation in size of the disk.

The non-guided breathing rate of the patient in the bore is also taken into account, by obtaining a reference breathing rate. The stress experienced in the context of an MRI examination means that the breathing rate just prior to the MRI examination is not likely to be a relaxed breathing rate. The reference breathing rate is an actual initial or current breathing rate and it may be assumed to be somewhat higher than for relaxed breathing of the user. However, it provided a baseline relative to which a target breathing rate (typically at a lower rate) can be set.

The stimulus provided by the output device then optimally matches both the initial breathing rate of the patient and the rate of the rhythmic clicking sounds of the MRI scanner bore.

The rhythm information thus typically comprises a click rate (or equivalently click period) at which audible clicks are generated by the magnetic resonance imaging system, wherein the target breathing rate is selected such that the click rate is an integer multiple of the target breathing rate. The clicking sounds for example have a frequency of around 1Hz to 10Hz.

The breathing rate is typically much slower, e.g. around 0.04Hz (2.4 bpm hence 25s per breath) to 0.5Hz (30 bpm hence 2s per breath). Thus, the click rate may be an integer multiple of the target breathing rate. Put another way, the target breathing period is selected to be an integer multiple of the click period.

Assuming a natural breathing frequency of a user of 1/6 Hz, the system will use a multiple of 6 for the clicking sound for the example of a 1Hz clicking frequency.

The target breathing rate is for example selected as the closest rate to the reference breathing rate for which the click rate is an integer multiple of the target breathing rate. Thus, the target breathing rate is as close to the reference breathing rate as possible. This may be appropriate if the user has been in the scanner for a while and has already adapted to a comfortable relaxed breathing rate.

The target breathing rate may be selected as the closest rate at or below the reference breathing rate for which the click rate is an integer multiple of the target breathing rate. Thus, the target breathing rate is as close to the reference breathing rate as possible but deliberately an equal or lower rate, to induce calm and relaxed breathing.

The target breathing rate may be selected as the closest rate at or below a fraction of the reference breathing rate and for which the click rate is an integer multiple of the target breathing rate. Thus, the target breathing rate can deliberately be a lower rate than the reference breathing rate, by at least a preset amount, to induce calm and relaxed breathing.

For example, with a clicking frequency of 1Hz and a reference breathing rate of 0.18Hz, for a fraction of 0.8, the fraction of the breathing rate becomes 0.144Hz, and the closest rate or below this fraction of the reference breathing rate is for an integer multiple of 7. Thus, the final target breathing rate is 0.143Hz.

The system may further comprise a prediction unit for predicting the rhythm information based on the magnetic resonance imaging apparatus characteristics and operating sequence, and for providing the rhythm information to the input. Thus, the rhythm information may be derived from the known characteristics of the MRI system and the operating sequence which is to be applied. This avoids the need to make any measurements.

Alternatively, or additionally, a microphone may be provided for sensing the rhythm information and providing the rhythm information to the input. Thus, the clicking sounds may be sensed. This sensing could be from a previous scan with the same settings (so that the rhythm information is learned over time) but more preferably it is in real time (so that the output device adapts to the actual sounds).

The breathing sensor for example comprises a chest strap. MRI compatible respiration belts are known. Alternatively, the breathing sensor may comprise a camera based respiration sensor.

The output device is for example adapted to provide a cyclic visual and/or audible and/or tactile output. By combining sensor modalities, the user experience may be enhanced. The clicking sounds become a part of the overall breathing guidance output, supplemented by the additional output from the output device.

The invention also provides a magnetic resonance imaging system, comprising:
a magnetic resonance imaging unit, such as a functional magnetic resonance imaging unit; and
the breathing guidance system as defined above.

The invention also provides a method of providing breathing guidance for guiding the breathing of a user during a magnetic resonance imaging procedure, such as a functional magnetic resonance imaging procedure, comprising:
receiving rhythm information relating to sounds generated by a magnetic resonance imaging system during a particular scanning procedure;
in advance of the imaging procedure, sensing breathing of the user determining a reference breathing rate of the user;
determining a target breathing rate for the user based on the rhythm information and the reference breathing rate, which target breathing rate is in synchronism with the generated sounds; and
providing a stimulus for guiding the user for guiding the user to breathe at the target breathing rate during the imaging procedure.

The rhythm information may comprise a click rate at which audible clicks are generated by the magnetic resonance imaging system, wherein determining a target breathing rate comprises selecting a target breathing rate such that the click rate is an integer multiple of the target breathing rate.

Selecting the target breathing rate may comprise setting a rate which is:
the closest rate to the reference breathing rate for which the click rate is an integer multiple of the target breathing rate; or
the closest rate at or below the reference breathing rate for which the click rate is an integer multiple of the target breathing rate; or
a fraction of the closest rate for which the click rate is an integer multiple of the target breathing rate.

The method may be implemented at least in part in software.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows an example of a magnetic resonance imaging system including a breathing guidance system;
Figs. 2A and 2B shows results of experiments to demonstrate the effectiveness of multimodal sensory stimuli in guiding breathing;
Fig. 3 shows a rhythmic sequence representing a rhythmic clicking sound and with markings to represent a first possible type of stimulus for breathing rate guidance;
Fig. 4 shows a rhythmic sequence representing a rhythmic clicking sound and with markings to represent a second possible type of stimulus for breathing rate guidance; and
Fig. 5 shows a method of providing breathing guidance.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a breathing guidance system for guiding the breathing of a user during a magnetic resonance imaging procedure. A target breathing rate is determined for the user which is in synchronism with the sounds, such as clicking sounds, generated by the MRI scanner. In this way, an improved signal to noise ratio is obtained for the scanned image by controlling the breathing of the user to be regular. The use of the clicking sounds as part of the breathing guidance system makes it easier for a user to maintain a certain breathing rate, since it is in synchronism with another rhythmical stimulus (which cannot be withheld from the user).

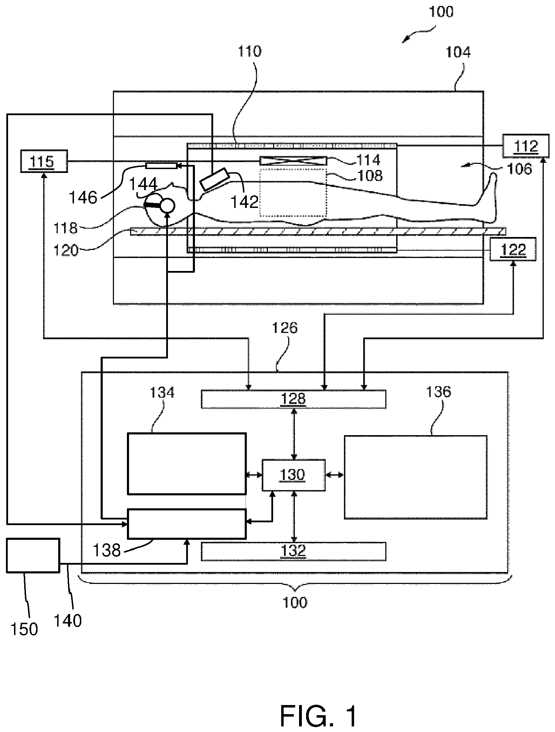

Fig. 1 shows an example of a magnetic resonance imaging system 100 with a magnet 104. The main magnet 104 is for example a superconducting cylindrical type magnet 104 with a bore 106 through it. The use of different types of magnets is also possible.

Within the bore 106 of the cylindrical magnet 104 there is an imaging zone 108 where the magnetic field is strong and uniform enough to perform magnetic resonance imaging.

Within the bore 106 of the magnet there is also a set of magnetic field gradient coils 110 forming a magnetic field gradient system which is used for acquisition of magnetic resonance data to spatially encode magnetic spins within the imaging zone 108 of the magnet 104. The magnetic field gradient coils 110 connected to a magnetic field gradient coil power supply 112. The magnetic field gradient coils 110 are intended to be representative.

Typically, magnetic field gradient coils 110 contain three separate sets of coils for spatially encoding in three orthogonal spatial directions. A magnetic field gradient power supply supplies current to the magnetic field gradient coils. The current supplied to the magnetic field gradient coils 110 is controlled as a function of time and may be ramped or pulsed. Adjacent to the imaging zone 108 is a radio-frequency coil 114, also referred to as radio frequency antenna system, for manipulating the orientations of magnetic spins within the imaging zone 108 and for receiving radio transmissions from spins also within the imaging zone 108. The radio frequency coil 114 may contain multiple coil elements.

The radio-frequency coil 114 is connected to a radio frequency transceiver 115. The radio frequency coil 114 and radio frequency transceiver 115 may be replaced by separate transmit and receive coils and a separate transmitter and receiver. It is understood that the radio- frequency coil 114 and the radio frequency transceiver 115 are representative.

The subject support 120 is attached to an optional actuator 122 that is able to move the subject support and the subject 118 through the imaging zone 108. In this way, a larger portion of the subject 118 or the entire subject 118 can be imaged. The transceiver 115, the magnetic field gradient coil power supply 112 and the actuator 122 are shown as being connected to a hardware interface 128 of computer system 126.

The computer 126 is further shown as including a processor 130 which is operable for executing machine-readable instructions. The computer 126 is further shown as comprising a user interface 132, computer storage 134 and (read only) computer memory 136 which are all accessible and connected to the processor 130.

The computer memory 136 may contain one or more pulse sequences which enable the processor 130 to acquire magnetic resonance data using the magnetic resonance imaging system 100, the magnetic resonance data being stored in the computer storage 134.

An imaging reconstruction module of the processor 130 is used to reconstruct magnetic resonance images, to be stored in the storage, using the acquired magnetic resonance data.

To the extent described above, the magnetic imagine system is standard.

The invention provides a breathing guidance system for guiding the breathing of a user during a magnetic resonance imaging procedure. The breathing guidance system has a main control unit 138 with an input 140 for receiving rhythm information relating to sounds generated by the magnetic resonance imaging system during a particular scanning procedure.

A breathing sensor 142 is for sensing breathing of the user and for determining a reference breathing rate of the user. This reference breathing rate is for example obtained before the start of the imaging procedure. For example, it may be monitored over a time period, e.g. of at least a minute, before the start of the imaging procedure.

This reference breathing rate is unlikely to match a relaxed rest breathing rate for the user, due to the stress of the experience.

However, the current or initial breathing rate is of interest, because it is the known starting point from which the user's breathing may need to be adapted. The target breathing rate should not be too far from this initial breathing rate because that may not be achievable for the user.

The reference breathing rate may adapt during the MRI imaging session for example if the actual breathing rate has changed (either because of extra stress, or because the patient has successfully followed the breathing guidance presented up till then).

The reference breathing rate may for example be regularly updated throughout the imaging session to reflect the current breathing rate. This adaptation may be low-pass filtered to prevent that there are very frequent changes of the target breathing rate presented to the user. The reference breathing rate may for example be updated with a current measured breathing rate, each time a new target breathing rate is calculated.

The control unit 138 determines a target breathing rate for the user based on the rhythm information and the reference breathing rate. This target rate is in synchronism with the generated sounds, namely the clicking sounds discussed above.

In another implementation, the breathing guidance system is more fully integrated with the MRI system, in which case the processor 130 is used to perform the necessary signal processing functions (a coupling between the control unit 138 and the processor 130 is shown in Fig. 1 to represent this option).

The breathing guidance system may thus be a standalone system for connection to, and use with, an existing magnetic imaging system, or it may be an integrated functionality of the MRI system.

The breathing guidance system has an output device 144, 146 for providing a stimulus for guiding the user to breathe at the target breathing rate. The output device may provide one ore more different types of stimulus to the user.

The output device may be an audio device 144 as shown (headphones) and/or a visual output device 146 such as a screen or set of lights visible to the user during the imaging procedure. Tactile output such as vibration may additionally be used.

Fig. 1 shows an input unit 150 for determining the rhythm information, in particular the frequency/rate (or equivalently period), of the MRI scanner bore sound clicks. In a first approach, this frequency may be predicted based upon the scanning protocol to be applied and the (known) characteristics of the scanner. The frequency is thus determined by a generative model, based on the MRI acquisition sequence and the MRI device characteristics such as field strength, gradient strength, shielding, etc. The model simulates the acoustic output of the MRI machine. The model can be constructed by experimentation. This information is provided as the input 140 to the control unit 138.

A microphone (not shown) may additionally or alternatively be used to determine an actually detected frequency of the sound clicks if this is not clear from the scanning protocol. This may also be part of a calibration process, but it may equally be performed in real time, so that once the procedure has started, suitable breathing guidance can be given after a short initial settling period.

The current breathing period of the patient (and hence the reference breathing period) is for example determined using an MRI-compatible strap around the ribs or waist. Alternatively a camera based respiration sensor may be used to determine the current breathing period.

The control unit 138 compares the clicking sound rate (or period) with the reference breathing rate (or period). It then calculates a target breathing rate that matches the reference breathing rate as closely as possible subject to the condition that there is an integer number of the clicking sound periods in the target breathing period.

In a first approach, the target breathing rate is selected such that the click rate is an integer multiple of the target breathing rate and the closest rate to the reference breathing rate.

In a second approach, the target breathing rate is again selected as the closest rate at the reference breathing rate, but at or below the reference breathing rate so that the target breathing rate during the scanning is deliberately the same or lower, to induce calm in the patient.

The target breathing rate may indeed be a fraction of the closest rate for which the click rate is an integer multiple of the target breathing rate. For example, the target breathing rate may have a period around 1.1 times the closest breathing rate (but still maintaining an integer number of click periods within the period of the target breathing rate) in order to lower the breathing rate of the patient. This is beneficial for relaxation and provides the opportunity to scan more slices of body tissue during one breathing period.

The multiple 1.1 is for example more generally in the range 1.05 to 1.2, while maintaining the required integer multiple relationship as explained above.

The output device may be a visual display with a bar or a disk that grows in size in a cyclic way synchronized with the target breathing rate.

An MRI scanner typically comes with a mirror set up which is placed close to the patient's eyes. This enables the patient to see a projection of large images from outside the scanner bore. This invention may make use of the same projection and mirror system.

A sound simulation may instead or additionally be used. For example, the sound of breaking waves has been found in previous studies to function well as a breathing guidance stimulation. In this way, the clicks and the wave sounds combine to create an overall stimulation, in which the clicks are no longer perceived as annoying, but form part of the breathing guidance function. A tactile stimulus may also be used.

Multiple stimulation sources can be combined in a rhythmically synchronous way. There is evidence that rhythmical synchronous combinations of sensory modalities help a patient to focus on the sensory stimulation, thereby facilitating a modification in breathing rate.

For example, it has been shown in studies that rhythmically synchronous signals enable improved perception of information. The article of R. van Ee et. al. "Multisensory congruency as a mechanism for attentional control over perceptual selection", J Neurosci 29: 11641-11649, 2009 shows that heteromodal congruency is an efficient means for the brain to identify signal relevance in the bombardment of sensory signals. This is understood by the existence of a machinery of neurons especially devoted to combine perceptual functions across sensory modalities. These neurons play an important role in perceptual control to influence conscious experiences.

Signal congruency may thus facilitate multimodal mechanisms of voluntary perceptual control, since there is more support for a particular percept when there is information from another sensory modality that is congruent with it. Research and experiments by the applicant have also shown that rhythmically synchronous visual and auditory stimulation is able to positively influence the adherence to instructions for breathing guidance. Moreover, the patient is more relaxed because breathing exercises themselves are relaxing, and the patient can focus on the stimulation instead of focusing on other stresses.

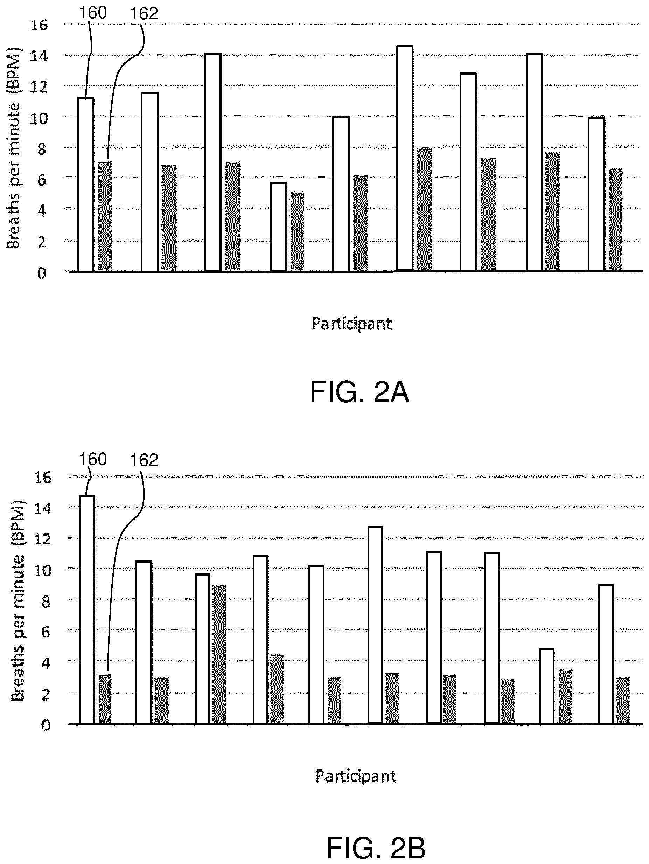

Figs. 2A and 2B shows results of the experiments mentioned above.

Fig. 2A shows the recorded breaths per minute for a set of 9 patients before the stimulus (the white bars 160) and with the stimulus (the solid bars 162). The stimulus is delivered by a rhythmically synchronous multisensory pillow, which provides a combination of vestibular and auditory stimulation. The vestibular stimulation is for example a slow repetitive motion of the head by a pillow that moves slightly up and down with a fixed frequency that resembles the breathing rate.

A target of 6 breaths per minute is set, and it can be seen that the patients adapt their breathing rate from a range 5 to 15 pm to a range 5 to 8 bpm. It also shows that a breathing rate reduction is more effective than a breathing rate increase.

Fig. 2B shows the recorded breaths per minute for a further set of 10 patients before the stimulus (the white bars 160) and with the stimulus (the solid bars 162). The stimulus is a rhythmically synchronous multisensory system, which provides a combination of visual and auditory stimulation.

A target of 3 breaths per minute is set, and it can be seen that the patients adapt their breathing rate from a range 5 to 15 pm to a range 3 to 9 bpm (with one outlier at 9 bpm and the others all in the range 3 to 4 bpm).

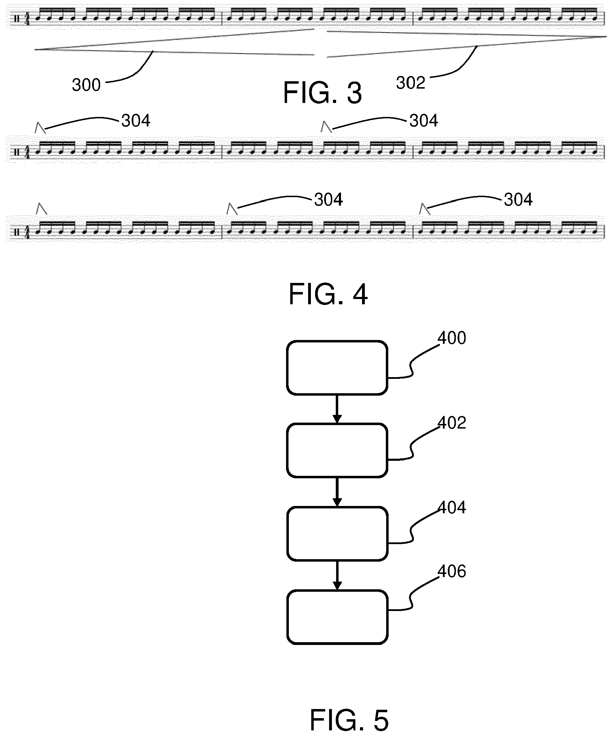

Fig. 3 shows a rhythmic sequence of sixteenth notes, to represent the rhythmic clicking sound of the MRI scanner. The crescendo marking 300 represents an increase or other progressive change in the accompanying stimulus (size or brightness of a displayed image, volume of an accompanying sound, color or other evolution of displayed image or object, etc.) and the decrescendo marking 302 represents a decrease or other gradual evolution in the accompanying stimulus.

Thus, the stimulus has a rate which matches the target breathing rate but the target breathing period can be clearly seen to be an integer multiple of the click period.

Fig. 4 shows accents 304 which represent a stimulus spike (sound or flash). These may be used to denote the start of the inhalation cycle and the start of the exhalation cycle. These may be used alone or in combination with the progressive stimulus of Fig. 3.

The target breathing rate may be adjusted by changing the positions of these accents 304 as shown in the bottom part of Fig. 4.

Fig. 5 shows a method of providing breathing guidance for guiding the breathing of a user during a magnetic resonance imaging procedure, such as a functional magnetic resonance imaging procedure. The method comprises:
in step 400, receiving rhythm information relating to sounds generated by a magnetic resonance imaging system during a particular scanning procedure;
in step 402, and in advance of the imaging procedure, sensing breathing of the user determining a reference breathing rate of the user;
in step 404, determining a target breathing rate for the user based on the rhythm information and the reference breathing rate, which target breathing rate is in synchronism with the generated sounds; and
in step 406, providing a stimulus for guiding the user for guiding the user to breathe at the target breathing rate during the imaging procedure.

As discussed above, embodiments make use of a control unit. The control unit can be implemented in numerous ways, with software and/or hardware, to perform the various functions required. A processor is one example of a control unit which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of control unit components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems. (optional)

If the term "adapted to" is used in the claims or description, it is noted the term "adapted to" is intended to be equivalent to the term "configured to".

Any reference signs in the claims should not be construed as limiting the scope.

## Claims

1. A breathing guidance system for guiding the breathing of a user during a magnetic resonance imaging procedure, comprising:
an input (140) for receiving rhythm information relating to sounds generated by a magnetic resonance imaging system during a particular scanning procedure;
a breathing sensor (142) for sensing breathing of the user and for determining a reference breathing rate of the user;
a processor (138;130) for determining a target breathing rate for the user based on the rhythm information and the reference breathing rate, the target rate being in synchronism with the generated sounds; and
an output device (144, 146) for providing a stimulus for guiding the user to breathe at the target breathing rate.

2. The breathing guidance system as claimed in claim 1, wherein the rhythm information comprises a click rate at which audible clicks are generated by the magnetic resonance imaging system, wherein the target breathing rate is selected such that the click rate is an integer multiple of the target breathing rate

3. The breathing guidance system as claimed in claim 1 or 2, wherein the target breathing rate is selected as the closest rate to the reference breathing rate for which the click rate is an integer multiple of the target breathing rate.

4. The breathing guidance system as claimed in claim 1 or 2, wherein the target breathing rate is selected as the closest rate at or below the reference breathing rate for which the click rate is an integer multiple of the target breathing rate.

5. The breathing guidance system as claimed in claim 1 or 2, wherein the target breathing rate is selected as the closest rate at or below a fraction of the reference breathing rate and for which the click rate is an integer multiple of the target breathing rate.

6. The breathing guidance system as claimed in any one of claims 1 to 5, further comprising a prediction unit (150) for predicting the rhythm information based on the magnetic resonance imaging apparatus characteristics and operating sequence, and for providing the rhythm information to the input.

7. The breathing guidance system as claimed in any one of claims 1 to 6, further comprising a microphone for sensing the rhythm information and providing the rhythm information to the input.

8. The breathing guidance system as claimed in any one of claims 1 to 7, wherein the breathing sensor comprises a chest strap.

9. The breathing guidance system as claimed in any one of claims 1 to 7, wherein the breathing sensor comprises a camera based respiration sensor.

10. The breathing guidance system as claimed in any one of claims 1 to 9, wherein the output device is adapted to provide a cyclic visual and/or audible and/or tactile output.

11. A magnetic resonance imaging apparatus, comprising:
a magnetic resonance imaging system such as a functional magnetic resonance imaging system; and
the breathing guidance system as claimed in any one of claims 1 to 10.

12. A method of providing breathing guidance for guiding the breathing of a user during a magnetic resonance imaging procedure, such as a functional magnetic resonance imaging procedure, comprising:
(400) receiving rhythm information relating to sounds generated by a magnetic resonance imaging system during a particular scanning procedure;
(402) in advance of the imaging procedure, sensing breathing of the user determining a reference breathing rate of the user;
(404) determining a target breathing rate for the user based on the rhythm information and the reference breathing rate, which target breathing rate is in synchronism with the generated sounds; and
(406) providing a stimulus for guiding the user for guiding the user to breathe at the target breathing rate during the imaging procedure.

13. The method as claimed in claim 12, wherein the rhythm information comprises a click rate at which audible clicks are generated by the magnetic resonance imaging system, wherein determining a target breathing rate comprises selecting a target breathing rate such that the click rate is an integer multiple of the target breathing rate.

14. The method as claimed in claim 13, comprising selecting the target breathing rate as:
the closest rate to the reference breathing rate for which the click rate is an integer multiple of the target breathing rate; or
the closest rate at or below the reference breathing rate for which the click rate is an integer multiple of the target breathing rate; or
a fraction of the closest rate for which the click rate is an integer multiple of the target breathing rate.

15. A computer program comprising computer program code means which is adapted, when said program is run on a computer, to implement the method of any one of claims 12 to 14.
